# FASCICULE DE BREVET EUROPEEN

(11) **EP 3 177 602 B1**
(45) Date de publication et mention de la délivrance du brevet: **26.10.2022**
(21) Numéro de dépôt: 15744276.5
(22) Date de dépôt: 17.07.2015
(51) Int. Cl.: C07D 323/04, C07D 323/06

(54) **PROCÉDÉ DE PRÉPARATION DU TRIOXANE**
VERFAHREN ZUR HERSTELLUNG VON TRIOXAN
METHOD FOR PREPARING TRIOXANE

(30) Priorité: 07.08.2014 FR 1457676
(43) Date de publication de la demande: 14.06.2017
(73) Titulaire: Arkema France, 92700 Colombes (FR)
(72) Inventeur: MONGUILLON, Bernard, F-64100 Bayonne (FR); LAFFITTE, Jean-Alex, F-64000 Pau (FR)
(74) Mandataire: Arkema Patent
(86) Numéro de dépôt international: PCT/FR2015/051968
(87) Numéro de publication internationale: WO 2016/020592

(56) Documents cités:
- WO-A1-95/15960
- WO-A1-2013/076286
- GB-A- 1 064 013

## Description

La présente invention se rapporte à un procédé de préparation du 1,3,5-trioxane, (désigné sous le nom de « trioxane » ci-après), à partir de formaldéhyde en présence d'un catalyseur acide spécifique permettant un rendement amélioré et une sélectivité élevée en trioxane tout en garantissant une économie d'énergie importante et une baisse des coûts de production du trioxane.

Le trioxane est très utilisé dans de nombreux domaines industriels et par exemple comme produit de départ dans la production de polyoxyméthylène (POM). Les POM ont de très bonnes caractéristiques physiques dont leur résistance élevée à la traction et aux chocs, une excellente stabilité dimensionnelle, de bonnes caractéristiques d'isolation électrique et une large plage de température d'utilisation. Actuellement, les POM sont utilisés dans de nombreuses industries telles que l'industrie automobile, le sport et l'électronique.

Le marché des POM étant en pleine expansion, il y a donc un besoin de produire le trioxane, qui est un précurseur des POM, avec un rendement et une sélectivité élevée, en grande quantité, de la manière la plus économique possible et sans nuire à l'environnement.

Le trioxane est habituellement produit par trimérisation du formaldéhyde, selon des techniques bien connues. Généralement, le trioxane est formé à partir d'une solution aqueuse de formaldéhyde, plus ou moins concentrée, en présence de catalyseur acide à des températures élevées. Le trioxane est alors séparé du milieu réactionnel par distillation. La vapeur de synthèse, renfermant outre le trioxane, encore du formaldéhyde, de l'eau et des sous-produits de réaction, peut être rectifiée dans une colonne d'enrichissement. La fraction obtenue riche en trioxane est alors soumise à une extraction et/ou toute méthode de séparation connue de l'homme du métier.

Par exemple, il est possible de procéder directement à l'extraction du trioxane après la distillation de celui-ci à l'aide d'un agent d'extraction non-miscible à l'eau. L'extraction, qui permet de récupérer le trioxane dans l'agent d'extraction, est généralement suivie d'une distillation de la phase organique, qui permet d'obtenir le trioxane pur, ou tout au moins de très grande pureté.

Cependant, les méthodes connues de l'homme du métier souffrent encore de nombreux inconvénients et les améliorations proposées n'ont pas réellement permis d'améliorer encore, ou d'optimiser le procédé de préparation du trioxane.

Le document GB949145 décrit la préparation du trioxane à partir de formaldéhyde en solution aqueuse en présence d'acides minéraux forts, par exemple, acide sulfurique, acide perchlorique et acide phosphorique; ou d'acides organique forts, tels que les acides sulfoniques aromatiques, particulièrement l'acide benzènesulfonique et ses homologues, par exemple, l'acide paratoluènesulfonique. Seul l'acide sulfurique est exemplifié dans ledit document.

Le document US2006/0058537 décrit un procédé de préparation du trioxane en présence de catalyseurs. Les catalyseurs sont des acides forts, par exemple l'acide sulfurique, l'acide trifluorométhanesulfonique, l'acide toluènesulfonique ou des échangeurs d'ions très acides. Il est également possible d'utiliser des zéolithes acides ou des hétéropolyacides. Dans ce document encore, seul l'acide sulfurique est exemplifié.

Le document FR1549133 décrit un procédé de préparation du trioxane en présence de catalyseurs acides. Parmi ces acides, de préférence l'acide sulfurique est utilisé, et par exemple l'acide phosphorique peut être utilisé. D'autres exemples de catalyseurs sont les sels acides, tels que l'hydrogénosulfate de potassium ou le chlorure de zinc, des acides sulfoniques aliphatiques et aromatiques, tels que l'acide p-toluènesulfonique ou l'acide 1-5-naphtalène-disulfonique, ou bien des échangeurs d'ions acides, par exemple, les résines échangeuses de cations du commerce portant des radicaux SO₃H. Seul l'acide sulfurique est exemplifié.

Le document WO2013/076286 décrit un procédé de préparation du trioxane en présence de divers catalyseurs acides et dans un solvant aprotique. Cependant, une étape de préparation du formaldéhyde dans un solvant aprotique allonge la durée du procédé. En outre, cette étape supplémentaire nécessite de modifier les équipements industriels déjà existants. De préférence, les catalyseurs utilisés sont les acides de Brønsted et les acides de Lewis. Le catalyseur est de préférence choisi dans le groupe constitué de l'acide trifluorométhanesulfonique, l'acide perchlorique, l'acide méthanesulfonique, l'acide toluènesulfonique et l'acide sulfurique ou leurs dérivés tels que les anhydrides ou les esters ou tout autre dérivé qui génère l'acide correspondant. Les acides de Lewis tels que le trifluorure de bore, le pentafluorure d'arsenic peuvent aussi être utilisés. Il est également possible d'utiliser un mélange de catalyseurs cités précédemment. Dans les exemples, l'acide sulfurique, l'acide trifluorométhanesulfonique, l'acide perchlorique et une résine échange d'ions sulfolane sont utilisés.

Le document GB1064013 décrit un procédé de préparation du trioxane en présence d'un émulsifiant acide comprenant un ou plusieurs acides alkylsulfoniques ou arylalkylsulfoniques ou alkylarylsulfoniques ou arylsulfoniques avec une ou plusieurs fonctions acide sulfonique. De préférence, les groupements alkyles des acides alkylsulfoniques contiennent de 14 à 16 atomes de carbone. Seuls lesdits acides alkylsulfoniques en C₁₄-C₁₆ sont exemplifiés.

Divers catalyseurs, acides inorganiques et organiques, ont été mentionnés tels que l'acide paratoluènesulfonique. Cependant, il présente l'inconvénient d'être sous forme de solide à température ambiante nécessitant ainsi une étape de dilution dans un solvant. Par ailleurs, seuls l'acide sulfurique, l'acide trifluorométhanesulfonique, l'acide perchlorique et une résine échangeuse d'ions sulfolane sont exemplifiés.

Le document WO 95/15960 divulgue un procédé de production en continu de trioxane à partir d'une solution aqueuse de formaldéhyde, en présence d'une résine échangeuse de cations comprenant un acide sulfonique tel que l'acide méthanesulfonique. Dans ce document, le solvant utilisé pour cette réaction est un solvant non miscible avec une solution aqueuse de formaldéhyde et miscible avec le trioxane. Les hydrocarbures aliphatiques ainsi que leurs dérivés halogénés sont cités.

Bien que plusieurs catalyseurs acides ont été cités dans les documents de l'art antérieur, et notamment les documents de l'art antérieur cités précédemment, l'acide sulfurique se présente aujourd'hui comme le catalyseur acide le plus largement utilisé pour la préparation du trioxane. Néanmoins, l'acide sulfurique présente l'inconvénient d'être corrosif et de générer des produits secondaires qui conduisent à une perte significative du rendement de production en trioxane. En effet, les principales réactions secondaires mises en jeu sont :
- la dismutation du formaldéhyde (CH₂O) en présence d'eau (H₂O) en acide formique (HCOOH) et en méthanol (CH₃OH), (réaction de Cannizzaro), selon le schéma suivant : 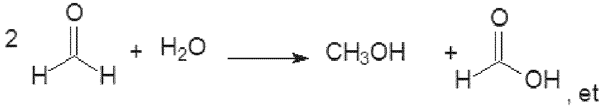
- la réaction équilibrée suivante, conduisant à la formation du formiate de méthyle (HCOOCH₃) à partir de l'acide formique et du méthanol formés précédemment :

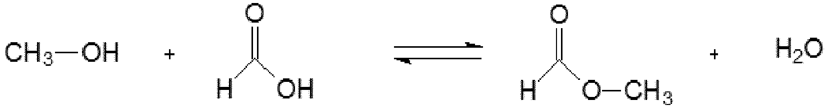

On peut également observer la réaction secondaire suivante qui génère du méthanol et du dioxyde de carbone (CO₂) : 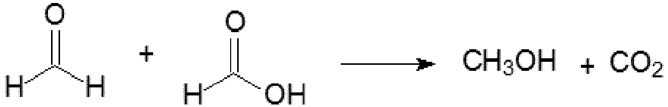 ainsi que, en milieu acide, la formation d'acide glycolique (HOCH₂COOH) :

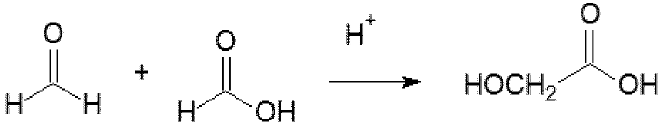

Il existe donc toujours un besoin de disposer d'une source simple, fiable, économique et respectueuse de l'environnement de préparation de formaldéhyde et ne nécessitant pas de grandes modifications industrielles. Il existe ainsi un besoin de disposer d'un procédé permettant d'améliorer le rendement et la sélectivité en trioxane par rapport aux méthodes connues de l'homme du métier; un procédé permettant également une diminution voire une élimination significative des produits secondaires. Il existe enfin un besoin de disposer d'un procédé de préparation du formaldéhyde utilisant un catalyseur acide moins nocif pour l'environnement, par rapport aux catalyseurs acides généralement utilisés.

La présente invention présente de nombreux avantages et notamment permet de remédier, en totalité ou au moins en partie, aux inconvénients présentés ci-dessus. D'autres avantages encore apparaitront à la lumière de la description de l'invention qui suit. L'invention concerne ainsi un procédé simple, efficace, et économique permettant de disposer d'une source de formaldéhyde sous forme de formaldéhyde trimérisé. Selon un premier aspect, l'invention concerne un procédé de trimérisation de formaldéhyde, et plus précisément un procédé de préparation du trioxane à partir du formaldéhyde, en milieu aqueux ou hydro-organique, ledit procédé comprenant au moins les étapes suivantes :
1) mise en contact de formaldéhyde avec un catalyseur comprenant au moins de l'acide méthanesulfonique ;
2) réaction de trimérisation dudit formaldéhyde en trioxane ; et
3) séparation du trioxane du milieu réactionnel ;
la teneur en eau dans le réacteur allant de 30% à 70% en poids, bornes incluses, par rapport au poids total du mélange réactionnel.

Selon un second aspect, la présente invention a pour objet l'utilisation d'un catalyseur comprenant de l'acide méthanesulfonique pour trimériser le formaldéhyde, et en particulier pour préparer du trioxane, ledit procédé de trimérisation étant réalisé en milieu aqueux ou hydro-organique et la teneur en eau dans le réacteur allant de 30% à 70% en poids, bornes incluses, par rapport au poids total du mélange réactionnel.

La présente invention a également pour objet l'utilisation d'un catalyseur comprenant de l'acide méthanesulfonique pour augmenter le rendement trimérisation du formaldéhyde, et plus particulièrement pour produire du trioxane, ledit procédé de trimérisation étant réalisé en milieu aqueux ou hydro-organique et la teneur en eau dans le réacteur allant de 30% à 70% en poids, bornes incluses, par rapport au poids total du mélange réactionnel.

La présente invention a enfin pour objet l'utilisation d'un catalyseur comprenant de l'acide méthanesulfonique pour augmenter la sélectivité en trioxane lors de la trimérisation du formaldéhyde, ledit procédé de trimérisation étant réalisé en milieu aqueux ou hydro-organique et la teneur en eau dans le réacteur allant de 30% à 70% en poids, bornes incluses, par rapport au poids total du mélange réactionnel.

Il a en effet été découvert de manière surprenante que l'utilisation d'un catalyseur comprenant de l'acide méthanesulfonique en tant que catalyseur acide en milieu aqueux permet d'améliorer notamment le rendement de la réaction de trimérisation du formaldéhyde, le rendement en trioxane et la sélectivité en trioxane. Par ailleurs, le catalyseur comprenant de l'acide méthanesulfonique est moins néfaste pour l'environnement que les catalyseurs acides généralement utilisés pour la trimérisation du formaldéhyde.

Sauf mention contraire, les pourcentages de matière mentionnés sont des pourcentages en poids.

On entend par « mélange réactionnel », le mélange comprenant tous les réactifs de départ. On entend par « milieu réactionnel », le mélange comprenant tous les produits formés au cours de la réaction de trimérisation du formaldéhyde, ainsi que tous les réactifs présents au début de la réaction et qui n'ont pas réagi.

L'invention a ainsi pour objet un procédé de trimérisation du formaldéhyde, et plus particulièrement de préparation du trioxane tel que défini précédemment.

Avantageusement, le procédé de trimérisation du formaldéhyde comprenant au moins les étapes 1) à 3) comme indiqué précédemment.

L'étape 2) de formation du trioxane est réalisée par réaction de trimérisation du formaldéhyde en présence d'au moins un catalyseur comprenant de l'acide méthanesulfonique. La réaction de trimérisation du formaldéhyde en trioxane peut être représentée par le schéma suivant :

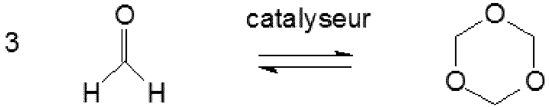

Cette étape de formation du trioxane est réalisée de préférence en milieu aqueux. Il est cependant également possible d'opérer en milieu solvant hydro-organique, c'est-à-dire dans un mélange eau/solvant organique, ledit solvant organique étant de préférence tel que défini ci-dessus. Les meilleurs résultats en termes de rendement, de sélectivité et de protection de l'environnement ont cependant été obtenus en réalisant le procédé de l'invention en milieu aqueux, et avantageusement en l'absence de solvant organique.

Le formaldéhyde (matière première) peut être utilisé sous toute forme connue de l'homme du métier, par exemple et le plus souvent soit en solution aqueuse soit sous forme solide, qui correspond alors à la forme appelée paraformaldéhyde. Les deux principales sources de formaldéhyde solide sont le paraformaldéhyde (oligomères du formaldéhyde) et les polyoxyméthylènes (polymères de formaldéhyde).

Pour les besoins du procédé de l'invention, le formaldéhyde est utilisé en solution aqueuse. Avantageusement, ladite solution aqueuse comprend le formaldéhyde, de l'eau et éventuellement des additifs. Avantageusement, la teneur en formaldéhyde va de 30% à 90% en poids, bornes incluses, par rapport au poids total de la solution aqueuse. De préférence, la teneur en formaldéhyde va de 30% à 70%, de préférence encore de 30% à 50% en poids, bornes incluses, par rapport au poids total de la solution aqueuse. De manière encore plus préférentielle, la teneur en formaldéhyde est d'environ 40 % en poids par rapport au poids total de la solution aqueuse.

Dans le réacteur, le formaldéhyde est introduit de telle sorte que la teneur en formaldéhyde va de 30% à 90% en poids par rapport au poids total du mélange réactionnel. De préférence, la teneur en formaldéhyde va de 30% à 70% en poids par rapport au poids total du mélange réactionnel. De préférence encore, la teneur en formaldéhyde va de 30% à 60% en poids par rapport au poids total du mélange réactionnel. Les plages précédentes s'entendent bornes incluses.

Le mélange réactionnel contenant de l'eau, celle-ci peut avantageusement provenir de la solution aqueuse comprenant le formaldéhyde, mais aussi éventuellement du catalyseur sous forme aqueuse tel que défini ci-après, ainsi que éventuellement d'additifs en solution aqueuse tel que définis ci-après. Il bien entendu possible d'ajouter de l'eau et/ou un autre solvant, par exemple un solvant organique dans le mélange réactionnel.

La teneur en eau dans le réacteur va de 70% à 30% en poids par rapport au poids total du mélange réactionnel, de préférence encore, de 70% à 50% en poids, bornes incluses par rapport au poids total du mélange réactionnel, et par exemple est égale à environ 60% en poids par rapport au poids total du mélange réactionnel.

Le mélange réactionnel comprend éventuellement des additifs tels que des agents anti-moussants, des agents anticorrosion, des tensioactifs, des stabilisants, et autres. Comme indiqué précédemment, les additifs précités peuvent éventuellement être sous forme de solution aqueuse.

La teneur en additif dans le milieu réactionnel va généralement de 0 à 15%, bornes incluses, en poids par rapport au poids total du mélange réactionnel. De préférence, la teneur en additif dans le mélange réactionnel va de 1 à 10% en poids, bornes incluses, par rapport au poids total du mélange réactionnel, et de préférence encore, de 1% à 5% en poids, bornes incluses, par rapport au poids total du mélange réactionnel.

Il a été découvert de manière tout à fait surprenante que l'utilisation d'au moins un catalyseur comprenant de l'acide méthanesulfonique permet d'augmenter significativement le rendement de trimérisation de formaldéhyde, ou de formation de trioxane, par rapport à l'utilisation de l'acide sulfurique qui est le catalyseur le plus couramment et largement utilisé pour préparer du trioxane à partir de formaldéhyde. Par ailleurs, le catalyseur comprenant de l'acide méthanesulfonique, grâce à ses propriétés non-oxydantes et biodégradables, rend le procédé moins nuisible pour l'environnement que les méthodes connues utilisant l'acide sulfurique.

Pour les besoins de la présente invention, on préfère utiliser un seul catalyseur comprenant de l'acide méthanesulfonique. Selon un mode de réalisation préféré, le catalyseur comprenant de l'acide méthane sulfonique est une solution aqueuse d'acide méthanesulfonique. Il est cependant possible d'utiliser de l'acide méthane sulfonique anhydre, connu sous le nom d'AMSA pour « Anhydrous Methane Sulphonic Acid » en langue anglaise, cette possibilité étant tout à fait adapté à un procédé de synthèse de trioxane mettant en œuvre du formaldéhyde en solution aqueuse.

Dans un autre mode de réalisation préféré, le procédé selon la présente invention met en œuvre de l'acide méthanesulfonique en solution aqueuse comme catalyseur de trimérisation du formaldéhyde, ce qui offre également l'avantage d'éviter une étape supplémentaire de mise en solution du formaldéhyde dans l'eau et/ou dans un solvant organique.

Comme indiqué précédemment, le catalyseur comprenant de l'acide méthanesulfonique peut être utilisé sous forme anhydre ou en solution aqueuse. De préférence, le catalyseur selon l'invention est en solution aqueuse plus ou moins diluée, ledit catalyseur étant dilué dans de l'eau, éventuellement en présence d'additifs.

Ainsi, lorsque le catalyseur comprend de l'acide méthanesulfonique en solution aqueuse, celui-ci peut être utilisé dans des concentrations qui peuvent aller de 0,1% à 99,9% en poids d'acide, bornes incluses, de préférence de 1% à 99% en poids, bornes incluses, de préférence de 30% à 95% en poids, bornes incluses, par rapport au poids total de la solution aqueuse d'acide méthanesulfonique. De préférence, la teneur en acide méthanesulfonique va de 50% à 80% en poids par rapport au poids total du catalyseur, et de manière encore plus préférée la teneur en acide méthanesulfonique est de 70% en poids par rapport au poids total du catalyseur. Par exemple, l'acide méthanesulfonique (AMS) à 70% en poids dans l'eau est commercialisé par la société ARKEMA.

Dans le mélange réactionnel, le catalyseur comprenant de l'acide méthanesulfonique sous forme anhydre ou en solution aqueuse est introduit de telle sorte que la teneur en acide méthanesulfonique, exprimé sous forme anhydre, va de 0,1% à 25% en poids, bornes incluses, par rapport au poids total du mélange réactionnel. De préférence, la teneur en acide méthanesulfonique va de 0,1% à 15% en poids par rapport au poids total du mélange réactionnel et de préférence encore, de 2% à 10% en poids, bornes incluses, par rapport au poids total du mélange réactionnel.

Selon un mode de réalisation, le catalyseur comprenant l'acide méthanesulfonique peut également comprendre un ou plusieurs autres acides, de préférence acides forts, de préférence encore acides minéraux forts, tels que ceux habituellement utilisés pour la réaction de trimérisation du trioxane, et, à titre d'exemples non limitatifs, l'acide sulfurique, l'acide phosphorique, l'acide paratoluènesulfonique, l'acide benzènesulfonique, les acides alkylsulfoniques autres que l'acide méthanesulfonique, et les mélanges de deux ou plusieurs d'entre eux, en toutes proportions, ainsi que tout autre catalyseur acide susceptible d'être utilisé pour connus comme catalyseurs de la trimérisation de formaldéhyde en trioxane.

De préférence, lorsque le catalyseur comprend de l'acide méthanesulfonique et un ou plusieurs autres catalyseurs acides, la teneur pondérale en acide méthanesulfonique est supérieure à la quantité du ou des autres acides présents dans le mélange, par exemple deux ou trois fois supérieure.

L'étape 2) de trimérisation est avantageusement réalisée par chauffage du mélange réactionnel comprenant ledit au moins un catalyseur, le formaldéhyde, un solvant, avantageusement l'eau et les éventuels additifs tels que décrits ci-dessus. La température de la réaction va généralement de 0°C à 200°C, de préférence de 50°C à 150°C, de préférence encore, de 70°C à 140°C, et de manière encore plus préférentielle, de 90°C à 130°C.

La réaction de trimérisation peut être effectuée à pression atmosphérique, sous pression ou sous dépression. L'homme du métier saura adapter la pression notamment selon la température et la concentration en formaldéhyde dans le mélange réactionnel. Généralement, la pression peut être fixée entre 0,1 et 10 bars (soit entre 0,1.10⁵ Pa et 10.10⁵ Pa). De préférence, la pression va de 0,1 à 5 bars (soit entre 0,1.10⁵ Pa et 5.10⁵ Pa). La pression dans le réacteur peut être réglée de telle sorte qu'elle soit comprise entre les bornes indiquées précédemment. De manière tout à fait préférée la réaction est conduite à pression atmosphérique.

La formation du trioxane par trimérisation du formaldéhyde en présence d'au moins un catalyseur comprenant de l'acide méthanesulfonique est réalisée pendant la durée nécessaire à la conversion la plus complète possible du formaldéhyde en trioxane. Cette durée peut aller de quelques minutes à plusieurs heures.

L'étape de formation du trioxane peut-être réalisée avec ou sans agitation, de préférence sous agitation. Lorsque qu'il y a agitation, celle-ci peut être apportée par toute méthode connue de l'homme du métier, par exemple, un agitateur magnétique, à pales, à hélice, ou tout autre méthode d'agitation couramment utilisé et connu pour ce type de réaction.

La réaction de trimérisation du formaldéhyde étant une réaction équilibrée, il est possible de déplacer l'équilibre réactionnel selon le principe de Le Châtelier pour favoriser la formation du trioxane. Ainsi, et selon un mode de réalisation préféré de l'invention, la formation du trioxane peut être grandement favorisée par exemple en retirant le trioxane formé du milieu réactionnel. Cette opération peut être effectuée selon toute méthode bien connue de l'homme du métier, par exemple par soutirage, et avantageusement par distillation au cours de la réaction.

Comme indiqué précédemment, le trioxane peut être soutiré au cours de la formation du trioxane. Selon un autre mode de réalisation, le trioxane est soutiré à l'issu de l'étape 2), c'est-à-dire après la formation du trioxane. Le procédé selon l'invention peut être réalisé en batch ou en continu. De manière particulièrement avantageuse, lorsque le procédé est conduit en continu, le trioxane est soutiré en continu, tout au long de la réaction, dès sa formation dans le milieu réactionnel. Lorsque le procédé est conduit en batch, le trioxane est séparé du milieu réactionnel au cours ou à l'issu de l'étape 2).

Comme indiqué précédemment, l'étape de séparation du trioxane peut être effectuée par toute méthode connue de l'homme du métier. Selon un mode de réalisation, l'étape de séparation du trioxane est réalisée par distillation. La distillation est, par exemple, une distillation simple, une distillation fractionnée, appelée également rectification, une distillation sur film mince, une distillation moléculaire, ou une distillation azéotropique qui peut être réalisée en présence d'un tiers solvant, généralement un alcool tel que méthanol ou l'éthanol.

L'étape de distillation peut être suivie d'une étape supplémentaire de purification bien connue de l'homme du métier utilisant une ou plusieurs des méthodes connues suivantes : évaporation, filtration, extraction, distillation, recristallisation, et autre.

Selon une autre mode de réalisation, l'étape de séparation du trioxane du milieu réactionnel comprend au moins les deux sous-étapes suivantes :
a) une étape d'extraction réalisée avantageusement en présence d'un agent d'extraction ;
b) et une étape de distillation pour séparer le trioxane et l'agent d'extraction.

L'étape d'extraction a) est avantageusement réalisée en présence d'un agent d'extraction. Un agent d'extraction est un composé peu ou non-miscible à l'eau qui va entrainer le trioxane avec lui. L'agent d'extraction doit bien dissoudre le trioxane. Les agents d'extractions qui peuvent avantageusement être utilisés sont des solvants organiques tels que ceux choisis parmi le benzène, le chlorure de méthylène, le chlorure d'éthylène, le chloroforme, le cyclohexane, le chlorobenzène, le diéthyléther, et les mélanges de deux ou plusieurs d'entre eux. De manière préférée, leur point d'ébullition est compris entre 40°C et 120°C ; de préférence, entre 60°C et 120°C.

L'étape d'extraction a) est suivie d'une étape de distillation b). La distillation peut être une distillation simple ou une distillation fractionnée, ou toute autre méthode de distillation déjà indiquée *supra.*

L'étape de distillation b) peut être suivie d'une étape supplémentaire de séparation telle qu'une évaporation, une filtration, une extraction, une distillation, et autres.

Selon un second aspect, la présente invention a pour objet l'utilisation d'un catalyseur comprenant de l'acide méthanesulfonique, pour réaliser la trimérisation du formaldéhyde, en particulier pour préparer du trioxane, plus particulièrement du trioxane par trimérisation de formaldéhyde, telle que définie ci-dessus. Selon un mode de réalisation préféré, le catalyseur mis en œuvre pour l'utilisation selon la présente invention est l'acide méthanesulfonique, et de manière tout à fait préférée l'acide méthanesulfonique en solution aqueuse.

La présente invention a également pour objet l'utilisation d'un catalyseur comprenant de l'acide méthanesulfonique pour augmenter le rendement de production du trioxane, plus particulièrement de production du trioxane par trimérisation de formaldéhyde, telle que définie ci-dessus. Selon un mode de réalisation préféré, le catalyseur mis en œuvre pour l'utilisation selon la présente invention est l'acide méthanesulfonique, et de manière tout à fait préférée l'acide méthanesulfonique en solution aqueuse.

La présente invention a enfin pour objet l'utilisation d'un catalyseur comprenant de l'acide méthanesulfonique pour augmenter la sélectivité en trioxane, plus particulièrement de sélectivité en trioxane obtenu par trimérisation de formaldéhyde, telle que définie ci-dessus. Selon un mode de réalisation préféré, le catalyseur mis en œuvre pour l'utilisation selon la présente invention est l'acide méthanesulfonique, et de manière tout à fait préférée l'acide méthanesulfonique en solution aqueuse.

Les exemples suivants illustrent l'invention sans l'intention d'en limiter la portée telle que conférée par les revendications annexées.

On réalise des essais de synthèse de 1,3,5-trioxane en introduisant dans un réacteur, chauffé à 105°C, du formaldéhyde en solution à 60% dans l'eau et le catalyseur acide qui est soit l'acide méthane sulfonique, commercialisé par la société Arkema (exemple selon l'invention), soit l'acide sulfurique (exemple comparatif).

Le 1,3,5-trioxane formé est extrait du milieu réactionnel puis purifié par distillation. Les résultats sont indiqués dans le Tableau 1.

**-- Tableau 1 --**

| **Référence** | **Selon l'invention** | **Comparatif** |
|---|---|---|
| Formaldéhyde | 60% aqueux | 60% aqueux |
| Catalyseur | AMS 5% | H₂SO₄ 5% |
| Température | 105°C | 105°C |
| Durée | 3 h | 3 h |
| Taux de conversion du formaldéhyde | 40% | 25% |
| Sélectivité en trioxane | 97,50% | 95% |

Ces résultats montrent que l'AMS permet un meilleur taux de conversion du formaldéhyde, et une meilleure sélectivité en 1,3,5-trioxane, comparativement à un autre catalyseur acide fort : l'acide sulfurique.

## Revendications

1. Procédé de trimérisation de formaldéhyde, et plus précisément un procédé de préparation du trioxane à partir du formaldéhyde, en milieu aqueux, ou hydro-organique, ledit procédé comprenant au moins les étapes suivantes :
1) mise en contact de formaldéhyde avec un catalyseur comprenant au moins de l'acide méthanesulfonique ;
2) réaction de trimérisation dudit formaldéhyde en trioxane ; et
3) séparation du trioxane du milieu réactionnel ;
la teneur en eau dans le réacteur allant de 30% à 70% en poids, bornes incluses, par rapport au poids total du mélange réactionnel.

2. Procédé selon la revendication 1, dans lequel la teneur en formaldéhyde va de 30% à 90% en poids, de préférence de 30 à 70% en poids, de préférence encore, de 30% à 60% en poids par rapport au poids total du mélange réactionnel, bornes incluses.

3. Procédé selon la revendication 1 ou la revendication 2, dans lequel la teneur en acide méthanesulfonique sous forme anhydre ou en solution aqueuse dans le mélange réactionnel, va de 0,1% à 25% en poids, bornes incluses, d'acide méthanesulfonique exprimé sous forme anhydre, par rapport au poids total du mélange réactionnel, de 0,1% à 15% en poids, bornes incluses, et de préférence encore, de 2% à 10% en poids, bornes incluses.

4. Procédé selon l'une quelconque des revendications précédentes, dans lequel l'étape 2) de trimérisation est réalisée à une température allant de 0°C à 200°C; de préférence, de 50°C à 150°C ; de préférence encore, de 70°C à 140°C, et de manière encore plus préférée, de 90°C à 130°C.

5. Procédé selon l'une quelconque des revendications précédentes, dans lequel la réaction de trimérisation du formaldéhyde est réalisée à une pression fixée entre 0,1.10⁵ Pa et 10.10⁵ Pa, de préférence entre 0,1.10⁵ Pa et 5.10⁵ Pa, et de préférence encore la pression réactionnelle est la pression atmosphérique.

6. Procédé selon l'une quelconque des revendications précédentes, qui est réalisé en continu, avec soutirage en continu du trioxane formé.

7. Utilisation d'un catalyseur comprenant de l'acide méthanesulfonique, pour réaliser la trimérisation du formaldéhyde, en particulier pour préparer du trioxane, plus particulièrement du trioxane par trimérisation de formaldéhyde, ledit procédé de trimérisation étant réalisé en milieu aqueux ou hydro-organique et la teneur en eau dans le réacteur allant de 30% à 70% en poids, bornes incluses, par rapport au poids total du mélange réactionnel.

8. Utilisation selon la revendication 7, dans laquelle le catalyseur est l'acide méthanesulfonique, et de manière préférée l'acide méthanesulfonique en solution aqueuse.

9. Utilisation d'un catalyseur comprenant au moins de l'acide méthanesulfonique pour augmenter le rendement de production du trioxane obtenu par trimérisation de formaldéhyde, ledit procédé de trimérisation étant réalisé en milieu aqueux ou hydro-organique et la teneur en eau dans le réacteur allant de 30% à 70% en poids, bornes incluses, par rapport au poids total du mélange réactionnel.

10. Utilisation d'un catalyseur comprenant au moins de l'acide méthanesulfonique pour augmenter la sélectivité en trioxane obtenu par trimérisation de formaldéhyde, ledit procédé de trimérisation étant réalisé en milieu aqueux ou hydro-organique et la teneur en eau dans le réacteur allant de 30% à 70% en poids, bornes incluses, par rapport au poids total du mélange réactionnel.

## Patentansprüche

1. Verfahren zur Trimerisierung von Formaldehyd und spezieller Verfahren zur Herstellung von Trioxan aus Formaldehyd in wässrigem oder wässrig-organischem Medium, wobei das Verfahren mindestens die folgenden Schritte umfasst:
1) Inkontaktbringen von Formaldehyd mit einem Katalysator, der zumindest Methansulfonsäure umfasst;
2) Trimerisierungsreaktion des Formaldehyds zu Trioxan und
3) Abtrennen des Trioxans aus dem Reaktionsmedium; wobei der Wassergehalt im Reaktor im Bereich von 30 bis 70 Gew.-%, wobei die Grenzen eingeschlossen sind, bezogen auf das Gesamtgewicht der Reaktionsmischung, liegt.

2. Verfahren nach Anspruch 1, wobei der Formaldehydgehalt im Bereich von 30 bis 90 Gew.-%, bevorzugt 30 bis 70 Gew.-%, weiter bevorzugt 30 bis 60 Gew.-%, bezogen auf das Gesamtgewicht der Reaktionsmischung, liegt, wobei die Grenzen eingeschlossen sind.

3. Verfahren nach Anspruch 1 oder Anspruch 2, wobei der Gehalt an Methansulfonsäure in wasserfreier Form oder in wässriger Lösung in der Reaktionsmischung im Bereich von 0,1 bis 25 Gew.-%, wobei die Grenzen eingeschlossen sind, Methansulfonsäure, ausgedrückt in wasserfreier Form, bezogen auf das Gesamtgewicht der Reaktionsmischung, von 0,1 bis 15 Gew.-%, wobei die Grenzen eingeschlossen sind, und weiter bevorzugt 2 bis 10 Gew.-%, wobei die Grenzen eingeschlossen sind, liegt.

4. Verfahren nach einem der vorhergehenden Ansprüche, wobei der Trimerisierungsschritt 2) bei einer Temperatur im Bereich von 0 °C bis 200 °C, bevorzugt von 50 °C bis 150 °C, weiter bevorzugt von 70 °C bis 140 °C und noch weiter bevorzugt von 90 °C bis 130 °C durchgeführt wird.

5. Verfahren nach einem der vorhergehenden Ansprüche, wobei die Formaldehydtrimerisierungreaktion bei einem zwischen 0,1.10⁵ Pa und 10.10⁵ Pa, bevorzugt zwischen 0,1.10⁵ Pa und 5.10⁵ Pa, festgelegten Druck durchgeführt wird und der Reaktionsdruck weiter bevorzugt Atmosphärendruck ist.

6. Verfahren nach einem der vorhergehenden Ansprüche, das kontinuierlich durchgeführt wird, wobei das gebildete Trioxan kontinuierlich abgezogen wird.

7. Verwendung eines Katalysators, der Methansulfonsäure umfasst, zur Durchführung der Trimerisierung von Formaldehyd, insbesondere zur Herstellung von Trioxan, spezieller von Trioxan durch Trimerisierung von Formaldehyd, wobei das Trimerisierungsverfahren in wässrigem oder wässrig-organischem Medium durchgeführt wird und der Wassergehalt im Reaktor im Bereich von 30 bis 70 Gew.-%, wobei die Grenzen eingeschlossen sind, bezogen auf das Gesamtgewicht der Reaktionsmischung, liegt.

8. Verwendung nach Anspruch 7, wobei es sich bei dem Katalysator um Methansulfonsäure und vorzugsweise Methansulfonsäure in wässriger Lösung handelt.

9. Verwendung eines Katalysators, der zumindest Methansulfonsäure umfasst, zur Erhöhung der Ausbeute der Produktion von durch Trimerisierung von Formaldehyd erhaltenem Trioxan, wobei das Trimerisierungsverfahren in wässrigem oder wässrig-organischem Medium durchgeführt wird und der Wassergehalt im Reaktor im Bereich von 30 bis 70 Gew.-%, wobei die Grenzen eingeschlossen sind, bezogen auf das Gesamtgewicht der Reaktionsmischung, liegt.

10. Verwendung eines Katalysators, der zumindest Methansulfonsäure umfasst, zur Erhöhung der Selektivität bezüglich durch Trimerisierung von Formaldehyd erhaltenem Trioxan, wobei das Trimerisierungsverfahren in wässrigem oder wässrig-organischem Medium durchgeführt wird und der Wassergehalt im Reaktor im Bereich von 30 bis 70 Gew.-%, wobei die Grenzen eingeschlossen sind, bezogen auf das Gesamtgewicht der Reaktionsmischung, liegt.

## Claims

1. Process for the trimerization of formaldehyde, and more specifically a process for preparing trioxane from formaldehyde, in an aqueous or aqueous-organic medium, said process comprising at least the following steps:
1) bringing formaldehyde into contact with a catalyst comprising at least methanesulfonic acid;
2) carrying out a reaction of trimerization of said formaldehyde into trioxane; and
3) separating the trioxane from the reaction medium;
the water content in the reactor ranging from 30% to 70% by weight, limits included, relative to the total weight of the reaction mixture.

2. Process according to claim 1, in which the formaldehyde content ranges from 30% to 90% by weight, preferably from 30% to 70% by weight, more preferably from 30% to 60% by weight relative to the total weight of the reaction mixture, limits included.

3. Process according to claim 1 or claim 2, in which the content of methanesulfonic acid in anhydrous form or in an aqueous solution in the reaction mixture ranges from 0.1% to 25% by weight, limits included, of methanesulfonic acid expressed in anhydrous form, relative to the total weight of the reaction mixture, from 0.1% to 15% by weight, limits included, and more preferably from 2% to 10% by weight, limits included.

4. Process according to any one of the preceding claims, in which the trimerization step 2) is carried out at a temperature ranging from 0°C to 200°C; preferably from 50°C to 150°C, more preferably from 70°C to 140°C, and even more preferably from 90°C to 130°C.

5. Process according to any one of the preceding claims, in which the formaldehyde trimerization reaction is carried out at a pressure set between 0.1·10⁵ Pa and 10.10⁵ Pa, preferably between 0.1·10⁵ Pa and 5.10⁵ Pa, and more preferably the reaction pressure is atmospheric pressure.

6. Process according to any one of the preceding claims, which is carried out continuously, with continuous withdrawal of the trioxane formed.

7. Use of a catalyst comprising methanesulfonic acid, for carrying out formaldehyde trimerization, in particular for preparing trioxane, more particularly trioxane by formaldehyde trimerization, said trimerization process being carried out in an aqueous or aqueous-organic medium and the water content in the reactor ranging from 30% to 70% by weight, limits included, relative to the total weight of the reaction mixture.

8. Use according to claim 7, in which the catalyst is methanesulfonic acid, and preferably methanesulfonic acid in aqueous solution.

9. Use of a catalyst comprising at least methanesulfonic acid for increasing the yield from production of trioxane obtained by formaldehyde trimerization, said trimerization process being carried out in an aqueous or aqueous-organic medium and the water content in the reactor ranging from 30% to 70% by weight, limits included, relative to the total weight of the reaction mixture.

10. Use of a catalyst comprising at least methanesulfonic acid for increasing the selectivity with respect to trioxane obtained by formaldehyde trimerization, said trimerization process being carried out in an aqueous or aqueous-organic medium and the water content in the reactor ranging from 30% to 70% by weight, limits included, relative to the total weight of the reaction mixture.
